# EUROPEAN PATENT APPLICATION

(11) **EP 1 475 124 A1**
(43) Date of publication of application: **10.11.2004**
(21) Application number: 03425296.5
(22) Date of filing: 09.05.2003
(51) Int. Cl.: A61M 25/06

(54) **Safety catheter**

(71) Applicant: Restelli, Sergio, 00100 Rome (IT); Righi, Nardino, 20047 Brugherio (Milano) (IT); Rossi, Roberto, 20151 Milano (IT)
(72) Inventor: Restelli, Sergio, 00100 Rome (IT); Righi, Nardino, 20047 Brugherio (Milano) (IT); Rossi, Roberto, 20151 Milano (IT)
(74) Representative: Petruzziello, Aldo

(57) **Abstract**

A safety catheter (1) comprises a body (10) with an axial channel (11) communicating with a sheath or cannula (12) for administration of fluids. A guide needle (3), supported by a needle-carrier (2) which can be inserted, through the channel (11) of the catheter body, into the sheath (12) to guide it during insertion into the body of a patient is provided. The catheter (1) is coupled, in a bayonet coupling relationship, to a safety device (100) associated with the guide needle (3), to place the guide needle (3) in a condition of safety after use of the catheter.

## Description

The present invention refers to a safety catheter for injection and administration of medicinal liquids into a cavity of a patient's body.

As is known, as well as for drainage of physiological fluids from the patient's body, catheters also serve for injection or administration of medicinal liquids into the patient's body. For this purpose, a catheter generally comprises a central body connected to a small tube or flexible sheath, commonly known as a cannula, designed to be inserted into the patient's body for administration of medicinal liquids. Connectors are provided in the catheter body for connection of the catheter to bottles of medicinal liquids or to other medical instruments, such as pumps for administration of and therapy with medicinal liquids.

The catheter body also has an aperture with an axial channel through which a guide-needle is inserted into the catheter sheath to guide it inside the patient's body.

Catheters according to the prior art have some drawbacks from the point of view of practicality of use due to the difficulty in assembling and removing the catheter body in the guide needle support.

Furthermore, catheters of the prior art present some drawbacks from a safety viewpoint. In fact, once the catheter has been used, the tip of the guide needle remains outside the catheter body or the entire guide needle is extracted from the catheter body to be sent for disposal.

It is obvious that said operation of extraction of the guide needle proves extremely hazardous, with the risk of injury to the user and to the personnel responsible for disposal. Furthermore, even in the case of the guide needle remaining inserted in the catheter, it must be considered that the point of the guide needle protrudes forward from the catheter body and in any case this leads to the risk of injury and accidental needle sticks.

Furthermore, the catheter body generally has a radial connector for coupling with special medical instruments. Said connector has a radial channel communicating with the axial channel. As a result, when the catheter body is not coupled to the guide needle, in order to use the radial connector it is necessary to obstruct the entrance to the axial channel destined to receive the guide needle body with a special stopper. This operation proves complex for the user and requires an additional element to be provided, such as the closure stopper.

The object of the present invention is to overcome the drawbacks of the prior art by providing a safety catheter that is practical and simple for the user to use.

Another object of the present invention is to provide such a safety catheter that is able to avoid the hazard and risk of accidental needle sticks.

Yet another object of the present invention is to provide such a catheter that is versatile and suitable to be applied to different types of safety guide needle existing on the market.

Yet another object of the present invention is to provide such a safety catheter that is cheap and easy to make.

These objects are achieved in accordance with the invention with the characteristics listed in appended independent claim 1.

Advantageous embodiments of the invention are apparent from the dependent claims.

The safety catheter according to the invention comprises a body with an axial channel communicating with a sheath or cannula for administration of fluids. Said catheter can be coupled to a guide needle supported by a needle-carrier. The guide needle can be inserted through the channel of the catheter body into the sheath to guide it during insertion into the body of a patient.

A main feature of the invention is represented by the fact that the catheter is coupled, in a bayonet coupling relationship, to a safety device associated with the guide needle, to put the guide needle into a condition of safety after use of the catheter.

Said solution affords two main advantages. A first advantage is provided by the bayonet coupling which allows easy assembly and removal of the catheter. A second advantage is provided by the safety device which allows the guide needle to be protected, after use of the catheter, avoiding injuries and accidental needle sticks.

The safety device advantageously comprises two sleeves which slide telescopically on the guide needle body to cover the guide needle in a position of safety.

The catheter advantageously has a second passage for introduction of liquid communicating with the axial channel and inside the channel of the catheter body there is provided a stopper of elastic material able to be perforated by the guide needle and return elastically to the initial condition to prevent the passage of liquid, so that the second passage of the catheter can be used even when the axial channel is not engaged by the guide needle, thus preventing the liquid from flowing out from the axial channel of the catheter.

Further characteristics of the invention will be made clearer by the detailed description that follows, referring to a purely exemplary and therefore non-limiting embodiment thereof, illustrated in the appended drawings, in which:
Figure 1 is a perspective view of the safety catheter according to the invention shown assembled and ready for use;
Figure 2 is an exploded perspective view of the catheter of Figure 1 in which the sheath is shown partially broken off;
Figure 3 is an axial sectional view of the body of the catheter according to the invention;
Figure 4 is a side view of a first external telescopic member of the safety device of the catheter according to the invention;
Figure 5 is a cross sectional view taken along the sectional plane V-V of Figure 4;
Figure 6 is a side view of a second internal telescopic member of the safety device of the catheter according to the invention;
Figure 7 is an axial cross sectional view along the sectional plane VII-VII of Figure 6;
Figure 8 is a side view of the catheter of Figure 1, in which the sheath is shown broken off;
Figure 9 is a cross sectional view of the catheter along the sectional plane IX-IX of Figure 8, in which the catheter body has not been sectioned;
Figure 10 is a side view of the safety device of the catheter according to the invention, in the safety position, in which the catheter body has been removed;
Figure 11 is an axial sectional view taken along the sectional plane XI-XI of Figure 10.

A safety catheter according to the invention is described with the aid of the figures.

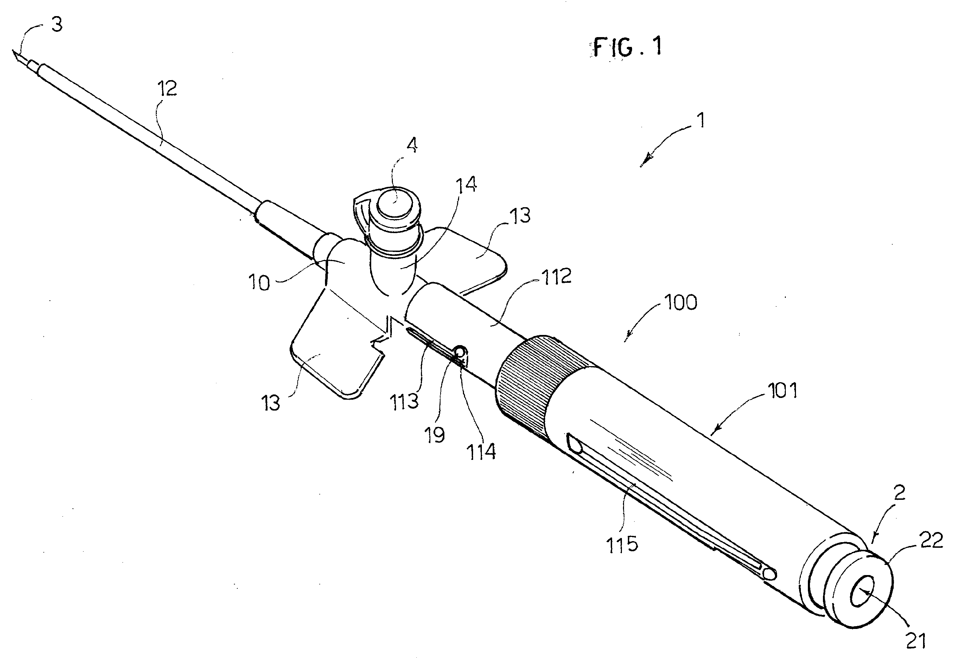

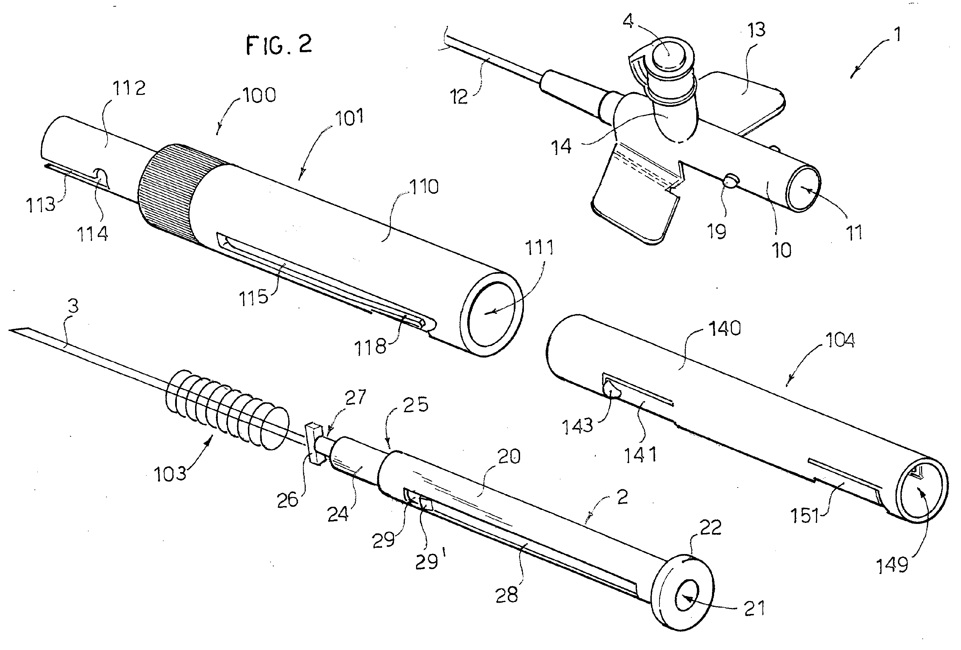

With reference in particular to Figures 1 and 2, a catheter proper is shown, denoted as a whole by reference numeral 1. The catheter 1 is engaged by a guide needle 3 supported by a needle-carrier 2. The guide needle 3 is made safe by means of a safety device 100, telescopic in shape, comprising a first external element 101, a second internal element 104 and a spring 103.

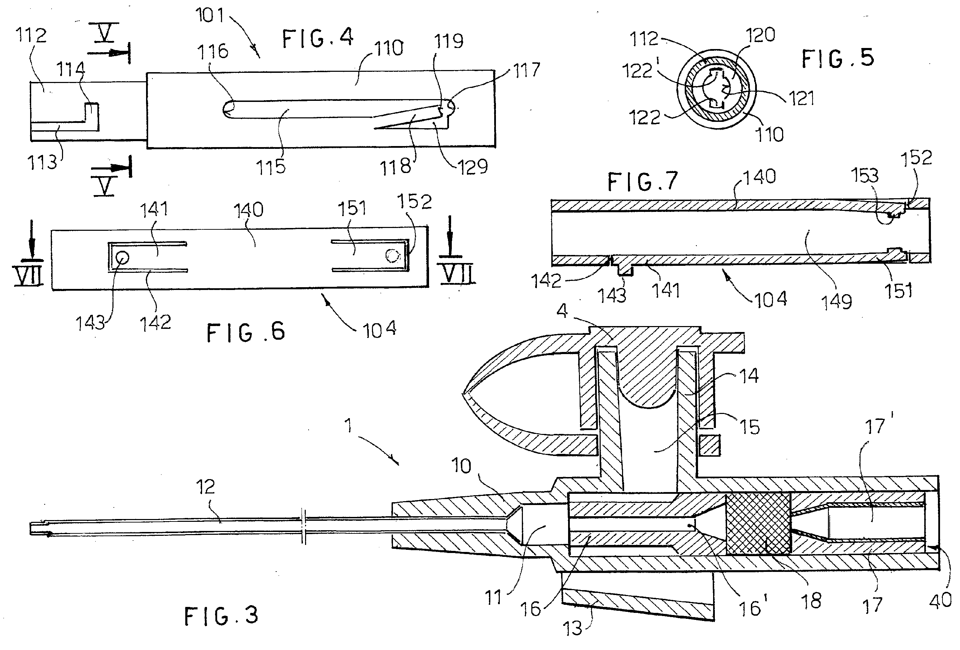

With reference in particular to Figure 3, the catheter 1 comprises a substantially cylindrical body 10 hollow on the inside so as to define an axial chamber 11 open at the front and rear.

At the head of the catheter body 10 there is mounted a sheath or cannula, in the form of a flexible tube 12, hollow on the inside and communicating at least with the front portion of the axial channel 11, for administration of the medicinal liquids.

Two flexible tongues 13, shaped as butterfly wings, protrude radially from the body 10 and can be bent by gripping by the operator from a divergent position (shown in the figures) to a convergent position.

The catheter body 10 also comprises a second connector 14 which protrudes radially therefrom and has a channel 15 communicating with the axial channel 11. The radial connector 14 is designed to be connected to a medical device for administration of a medicinal liquid for therapy. Said radial connector 14 is closed by means of an openable safety stopper 4.

In this manner, the catheter 1 provides two passages for introduction of liquids. A first passage consisting of the axial channel 11 and a second passage consisting of the radial channel 15.

As shown in Figure 3, a first shaped guide 16, a second shaped guide 17 and a stopper 18 disposed between the two guides 16 and 17 are disposed inside the axial channel 11 of the catheter body. The stopper 18 is disposed upstream with respect to the radial channel 15 of the second passage. The first and second guide 16, 17 have respective axial channels 16', 17' suitably tapered to guide insertion of the guide needle 3. It should be noted that the rear surface 40 of the second guide 17 is internal with respect to the rear surface of the catheter body 10. In this manner the surface 40 forms an annular abutment surface.

The stopper 18 is formed as a cylindrical block of resilient material, such as rubber or the like, which is able to prevent the passage of liquids and at the same time is able to be pierced by the guide needle 3, so that the guide needle 3 is guided through the guides 17 and 16 inside the sheath 12. Once the guide needle 3 has been extracted from the catheter body, the resilient material of the stopper 18 returns elastically to the initial condition and thus prevents the passage of liquids from the channel 16' of the first guide to the channel 17' of the second guide.

In this manner, the second passage of the catheter, that is to say the radial connector 14, can be used, without the need to insert the guide needle 3 into the axial channel 11 of the catheter, without the risk that the medicinal liquid administered might flow out from the rear of the catheter body 10 through the axial channel 11 of the catheter.

Returning to Figures 1 and 2, at the rear end of the catheter body 10 two nibs or tongues 19 which protrude radially outward in diametrically opposite positions are provided.

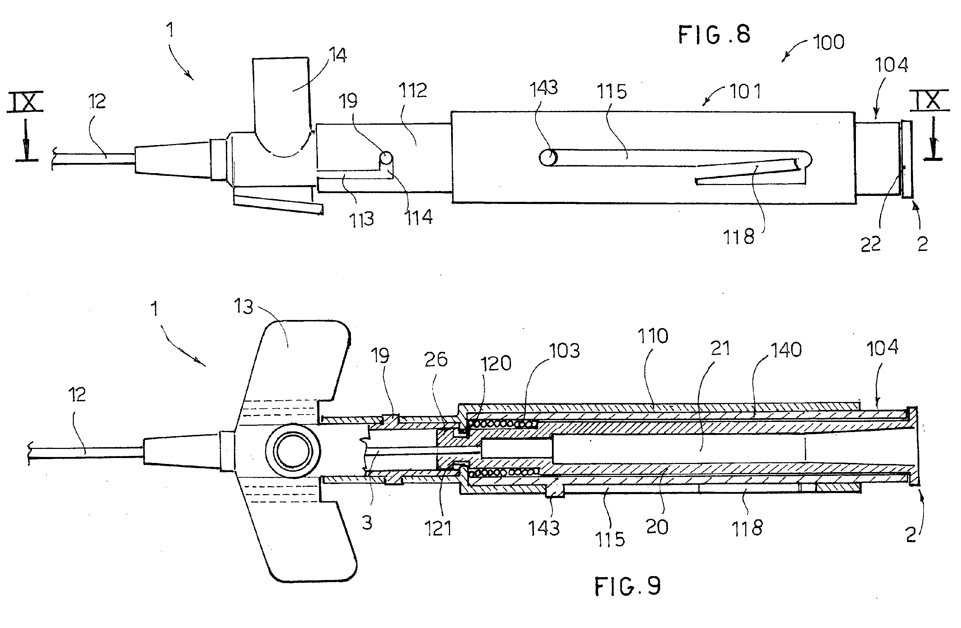

With reference to Figure 2, the needle-carrier 2 comprises a substantially cylindrical body 20 hollow on the inside and defining an axial channel 21 (Figure 9). As shown in Figure 9, the axial channel 21 has a tapered or truncated conical portion 23 which forms a standard type connector, for connection of medical accessories of the catheter for introduction of medicinal fluids.

The guide needle 3 is inserted from the rear into the axial channel 11 of the catheter body to enter the sheath 12 and guide it, during insertion into the body of the patient, for administration of medicinal fluids. In this manner, a first medicinal fluid can be injected from the rear connector 23 of the needle-carrier, through the guide needle 3, into the patient's body and a second medicinal liquid can be injected from the radial connector 14 of the catheter body, through the cannula 12, into the patient's body, passing peripherally around the outer surface of the guide needle 3.

The body 20 of the guide needle has at its rear end a disc-shaped flange 22 which protrudes radially therefrom.

In the front end of the body 20 of the needle-carrier there is provided a tang 24 with a smaller diameter than the body, so as to define an annular abutment surface 25. In front of the tang 24 there is provided a locking key 26 shaped as a small parallelepiped block. An annular narrowing 27 is defined between the locking key 26 and the tang 24.

On the outer side surface of the body 20 of the needle carrier there are formed two longitudinal grooves 28 disposed in diametrically opposite positions, which extend for a good part of the length of the body 20. Each groove 28 has at its front end a stopping seat 29 defined by a substantially tapered tooth 29'.

The safety device 100 is described hereunder with reference also to Figures 4-7. The safety device 100 comprises a first outer sleeve 101, a second inner sleeve 104 and a spiral spring 103.

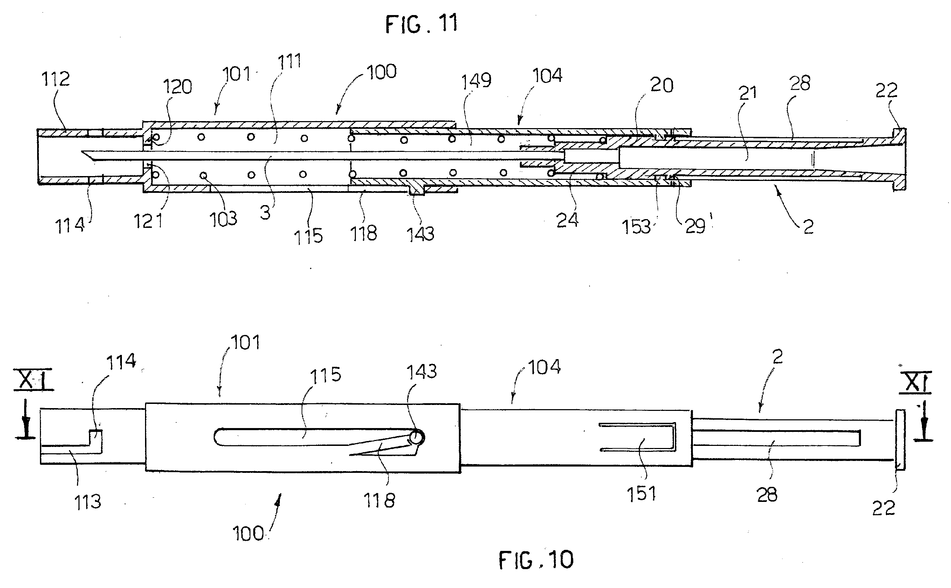

As also shown in Figure 4, the first sleeve 101 has a substantially cylindrical body 110 hollow on the inside so as to define an axial chamber 111 (Figure 11) open at the front and rear.

The first sleeve 101 has at the front a tang 112 with a smaller diameter than the body 110. The inside diameter of the tang 112 of the first sleeve is slightly greater than the outside diameter of the rear part of the catheter body 10, so that the rear part of the catheter body can be inserted inside the tang 112.

In the side part of the tang 112, at the front, two substantially L-shaped slots 113, disposed in diametrically opposite positions, are formed. Each slot 113 ends in a stopping seat 114. In this manner the slots 113 can be engaged by the nibs 19 of the catheter body, in a bayonet coupling relationship. As a result, to fix the catheter 1 to the sleeve 101, the nibs 19 must be inserted in the slots 113 and when the nibs 19 reach the end of their stroke, the catheter body must be rotated with respect to the sleeve, so that the nibs engage in the stopping seats 114 preventing any axial movement of the catheter 1 with respect to the sleeve 101.

A longitudinal slot 115 delimited by a front abutment surface 116 and a rear abutment surface 117 is provided in the body 110 of the first sleeve. An elastic tongue 118 is disposed obliquely in the rear part of the longitudinal slot 115.

The elastic tongue 118 has a rear abutment end 119 which is opposed to the rear abutment surface 117 of the slot 115 so as to define a stopping surface. The flexible tongue 118 is defined by a longitudinal cut 129 in the side wall of the body 110 of the first sleeve, so as to be able to bend elastically inside said longitudinal cut 129 and return elastically to the oblique starting position.

Clearly, even if not shown in the figures, two longitudinal slots 115 disposed in diametrically opposite positions are provided in the first sleeve 101.

As shown in Figure 5, between the body 110 and the tang 112 of the first sleeve, there is provided on the inside a transverse abutment flange 120 which has a central hole 121 communicating with two side openings 122, 122' disposed in diametrically opposite positions. The diameter of the hole 121 is smaller than the length of the parallelepiped locking key 26 of the needle-carrier, whilst the distance between the ends of the apertures 122, 122' is greater than the length of the locking key 26.

In this manner, if the locking key 26 is in register with the openings 122, 122', it can pass through the stop flange 120. Otherwise the locking key 26 remains locked in abutment against the stop flange 120.

As shown also in Figures 6 and 7, the second sleeve 104 has a substantially cylindrical body 140, hollow on the inside so as to define an axial chamber 149 open at the front and at the rear. The inside diameter of the body 140 of the second sleeve is slightly greater than the outside diameter of the body 20 of the needle carrier, so that the second sleeve 104 can slide axially on the needle carrier. Furthermore, the outside diameter of the second sleeve is slightly smaller than the inside diameter of the body 110 of the first sleeve, so that the first sleeve 101 can slide axially on the second sleeve 104.

In the side wall of the body 140 of the second sleeve there is provided at the front a longitudinal flexible tongue 141 defined by a substantially U-shaped cut 142, so as to be able to bend elastically towards the inside of the second sleeve. A nib 143 which protrudes radially outward is provided on the longitudinal tongue 141. The nib 143 is able to engage slidably in the longitudinal slot 115 of the first sleeve.

Clearly, if two slots 115 in diametrically opposite positions are provided in the first sleeve, accordingly two tongues 141 with respective nibs 143 disposed in diametrically opposite positions will be provided in the second sleeve.

Again in the side wall of the body 140 of the second sleeve, two flexible longitudinal tongues 151 disposed in diametrically opposite positions are provided. Each rear tongue 151 is defined by a substantially U-shaped cut 152, so as to be able to bend elastically toward the inside and toward the outside of the second sleeve. On each rear longitudinal tongue 151 there is provided a nib 153 which protrudes radially inward. The nib 153 is able to engage slidably in the longitudinal groove 28 in the body 20 of the needle carrier.

Assembly of the safety device 100 of the catheter 1 and the relative guide needle 3 with the needle carrier 2 is described hereunder, by way of example, with reference also to Figures 8 and 9.

Initially the spring 103 is disposed around the front tang 24 of the needle carrier 2 with one end of the spring 103 in abutment against the annular abutment surface 25 of the needle carrier 2 and the other end of the spring in abutment against the locking key 26.

Subsequently, the second sleeve 104 is inserted into the first sleeve 101, so that the nib 143 of the front tongue 141 of the first sleeve engages in the longitudinal slot 115 of the second sleeve. Then the second sleeve is made to slide inside the first sleeve until the nib 143 abuts against the front abutment surface 116 of the longitudinal slot 115 of the first sleeve. The front end of the second sleeve 104 abuts against the flange 120 of the first sleeve 101 and the rear end of the second sleeve protrudes rearward from the first sleeve.

At this point the catheter 1 is mounted in the head 112 of the first sleeve. The nibs 19 of the catheter are made to slide in the slots 113 of the top tang 112 of the first sleeve, until they reach the end of stroke. A rotation of the catheter with respect to the first sleeve is then performed and thus the nibs 19 of the catheter engage in the stopping slots 114 of the first sleeve, in a bayonet coupling relationship. In this situation any axial movement of the catheter with respect to the first sleeve is prevented.

Lastly, the needle carrier 2 provided with the spring 103 and the needle 3 is inserted axially from the rear into the second sleeve 104. By appropriately turning the needle-carrier 2, the locking key 26 of the needle carrier is brought into register with the apertures 122, 122' of the internal flange 120 of the first sleeve. In this situation, as shown in Figure 9, the needle-carrier can be further pushed inside the safety device 100 so that the key 26 passes beyond the flange 120.

One end of the spring 103, on the other hand, remains in abutment against the flange 120 and thus the spring 103 remains compressed between the flange 120 of the first sleeve and the abutment surface 25 of the needle carrier. Consequently, the needle-carrier 2 is rotated with respect to the first sleeve 101, so that the key 26 is no longer in register with the apertures 122, 122' and abuts against the flange 120 of the first sleeve, thus retaining the needle carrier inside the second sleeve 104.

In this situation the rear flange 22 of the needle-carrier abuts against the rear end of the second sleeve 104. Any axial movement of the needle-carrier with respect to the safety device 100 and against the biasing action of the spring 103 is thus prevented.

In this situation the needle 3 passes axially through the front part of the first sleeve 101 to enter into the channel 11 of the catheter wherein it is guided by the rear guide 17, pierces the stopper 18 and is guided by the front guide 16 inside the catheter sheath 12. The catheter is thus ready for use, with the guide needle 3 inserted in the sheath 12.

Operation of the catheter and of the safety device 100 is described below with particular reference also to Figures 10 and 11.

After having used the catheter for the treatment, the operator, holding the safety device 100 still, effects a rotation of the rear flange 22 of the needle-carrier. As a result the needle-carrier 2 rotates with respect to the first sleeve 101 until the key 26 of the needle-carrier comes into register with the apertures 122, 122' of the flange 120. In this situation, it should be noted that the nibs 153 of the rear tongues 151 of the second sleeve engage in the respective longitudinal grooves 28 of the needle-carrier.

At this point, the spring 103, which was compressed, is released and pushes the needle carrier 2 axially rearward. The needle-carrier 2 then performs a guided axial movement of withdrawal, wherein the nibs 153 of the rear tongues 151 of the second sleeve slide in the respective longitudinal slots 28 of the needle carrier until they reach the end of stroke, engaging in the locking seat 29 of the needle-carrier. In this situation any axial movement of the needle-carrier with respect to the second sleeve 104 is prevented.

As a result, again through the action of the spring 103, the needle-carrier 2 also pulls with it the second sleeve 104 which performs a guided axial movement of withdrawal, wherein the nib 143 of the front tongue 141 slides in the slot 115 of the first sleeve, until it reaches the end of stroke in abutment against the abutment surface 117. During sliding of the nib 143, the tongue 18 bends in the cut 129 and then returns elastically into an oblique position, so that its abutment surface 119 blocks the nib 143, preventing any axial movement of the first sleeve 101 with respect to the second sleeve 104.

At this point, as shown in Figure 11, the needle is completely protected by the first and the second sleeve 101, 104. The operator, acting on the bayonet coupling, then releases the catheter 1 from the first sleeve 101, first performing a rotation of the catheter with respect to the first sleeve, so that the nibs 19 of the catheter are released from the seats 114, and then a traction of the catheter with respect to the first sleeve, so that the nibs 19 slide in the slots 113 for total release. In this manner the catheter 1 is removed from the safety device 100 and the guide needle 3 remains protected inside the safety device 100 avoiding possible injuries and accidental needle sticks.

Numerous variations and modifications of detail within the reach of a person skilled in the art can be made to the present embodiment of the invention whilst in any case remaining within the scope of the invention as set forth in the appended claims.

## Claims

1. A safety catheter (1) comprising a body (10) with an axial channel (11) communicating with a sheath or cannula (12) for administration of fluids, said catheter (1) being able to be engaged by a guide needle (3) supported by a needle-carrier (2), said guide needle (3) being introducible, through the channel (11) of the catheter body, into the sheath (12) to guide it during insertion into a patient's body, **characterised in that** said catheter (1) is coupled, in a bayonet coupling relationship, to a safety device (100) associated with said guide needle (3) to cover it in a safety position.

2. A catheter according to claim 1, **characterised in that** said safety device (100) comprises a first sleeve (101) and a second sleeve (104) mounted telescopically slidably inside the first sleeve (101) and over the needle-carrier body (2), so that, when the guide needle (3) is in the safety position, it is covered by said first and second sleeve (101, 104).

3. A catheter according to claim 1 or 2, **characterised in that** said bayonet coupling provides a pair of nibs (19) mounted in diametrically opposite positions on the outer side surface of the catheter body (10) which engage in a respective pair of substantially L-shaped slots (113) formed in diametrically opposite positions at the front end of said first sleeve (101), each of said slots (113) ending in a locking seat (114) able to block said nibs (19) of the catheter.

4. A catheter according to any one of the preceding claims, **characterised in that** said catheter comprises a second connector (14) with a second channel (15) for introduction of fluids communicating with an axial channel (11) of the catheter body and there is provided upstream of the second channel (15) a stopper (18) of elastic material, able to be pierced by said guide needle (3) and return elastically to the initial state, when the guide needle (3) is extracted, so as to prevent the passage of liquids.

5. A catheter according to claim 4, **characterised in that** said stopper (18) is disposed between two shaped guides (16, 17) able to guide axial insertion of the guide needle (3) in the cannula (12).

6. A catheter according to any one of claims 2 to 5, **characterised in that** said safety device (100) comprises spring means (103) disposed under compression between an internal flange (120) of said first sleeve (101) and an abutment surface (25) of said needle-carrier.

7. A catheter according to claim 6, **characterised in that** said flange (120) has an axial hole (121) communicating with two radial apertures (122, 122') and at the front of said needle-carrier (2) there is provided a locking key (26) able to be inserted though the apertures (122, 122') of the flange to abut against the flange (121) so as to block the axial movement of withdrawal of the needle-carrier (2) with respect to the first sleeve (101) against the action of the spring (103).

8. A catheter according to any one of claims 2 to 7, **characterised in that** said first sleeve (101) has a longitudinal slot (115) with an elastic pawl (118) and said second sleeve (104) has an external nib (143) protruding radially outward to engage in said longitudinal slot, said external nib (143) being locked by the elastic pawl (118) when the guide needle is in the safety position.

9. A catheter according to any one of claims 2 to 8, **characterised in that** said needle-carrier has a pair of longitudinal grooves (28) disposed in diametrically opposite positions and provided with respective stop seats (29) and said second sleeve (104) has a pair of internal nibs (153) disposed in diametrically opposite positions and protruding radially inward inside the second sleeve, each internal nib (153) being able to engage in the respective stop seat (29) when the guide needle (3) is in the safety position.

10. A catheter according to claims 9 and 10, **characterised in that** said external nib (143) and said internal nibs (153) of the second sleeve are mounted on respective flexible tongues (141, 151) obtained by means of substantially U-shaped cuts in the body of the second sleeve.
